(19)

**Europäisches Patentamt**
**European Patent Office**
**Office européen des brevets**

(11)  **EP 3 793 493 B1**

(12) **EUROPEAN PATENT SPECIFICATION**

(45) Date of publication and mention
of the grant of the patent:
**23.08.2023  Bulletin 2023/34**

(21) Application number: **20720060.1**

(22) Date of filing: **24.04.2020**

(51) International Patent Classification (IPC):
*A61F 5/01* *(2006.01)*          *A61F 2/64* *(2006.01)*
*A61F 2/68* *(2006.01)*          *A61F 2/74* *(2006.01)*

(52) Cooperative Patent Classification (CPC):
**A61F 5/0123; A61F 2/64; A61F 2/68; A61F 2/741;**
**A61F 5/0125;** A61F 2005/0132

(86) International application number:
**PCT/EP2020/061408**

(87) International publication number:
**WO 2020/216876 (29.10.2020 Gazette 2020/44)**

(54) **WEARABLE ASSISTIVE DEVICE**

ASSISTIERENDE WEARABLE-VORRICHTUNG

DISPOSITIF D'ASSISTANCE PORTABLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **26.04.2019  EP 19171468**

(43) Date of publication of application:
**24.03.2021  Bulletin 2021/12**

(73) Proprietor: **MyoSwiss AG**
**8004 Zürich (CH)**

(72) Inventors:
• **KOGINOV, Gleb**
  **8045 Zürich (CH)**

• **SCHMIDT, Kai**
  **8047 Zürich (CH)**

(74) Representative: **Vossius & Partner**
**Patentanwälte Rechtsanwälte mbB**
**Siebertstrasse 3**
**81675 München (DE)**

(56) References cited:
**US-A- 5 547 464           US-A1- 2006 206 043**
**US-A1- 2010 056 970       US-A1- 2018 000 623**
**US-A1- 2018 193 179**

**Description**

The present invention relates to a wearable assistive device, as defined in the claims.

Wearable assistive devices are well known. They help a user to move, for example where a user is impaired due to a disease, an accident or where an exceptionally high load is to be moved by a user.

Wearable assistive devices can be implemented as purely passive assistive devices or as active assistive devices having active components such as motors, batteries for the motors, controls, sensors and so forth as well as having passive components such as tendons, ligaments and so forth attached to a limb so as to support movement by exerting a force that helps to move or stabilize the limb as necessary.

Where a wearable assistive device is used by a patient, it often is considered desirable that it can be worn for prolonged periods so as to enable a user to participate in normal social activities.

While a minority of users, in particular those that had accidents during skiing or other sports activities, may even take pride in wearing assistive devices as a sign of being particularly tough and risk-taking, a majority of users will prefer that the assistive devices is unobtrusive. This holds in particular for long-term users that need the devices day in and day out. Given the size of motors and batteries as well as the size of the mechanical arrangement needed to assist movement, hiding a wearable assistive device in use is a particular problem if the assisting device is an active device. For example, a typical wearable assistive device has several parts that will be attached to a limb proximal and distal of a joint and will then be actuated so as to assist movement.

One example of a dynamic orthotic knee extension assist device is known from US 4,100,918. The known dynamic orthotic knee extension assist device is intended for paraplegic patients and others needing such a device. Cuffs adapted to be secured to a patient's thigh are provided along with mounting means adapted to be secured to a patient's leg below the knee. An elongated elastic member is provided with a means for removably mounting each end thereof to the cuff means at a predetermined tension. Also provided is a means for pivotally securing the elastic means intermediate its ends to the mounting means. A lateral knee brace is rigidly disposed between the cuff and the mounting means. The elastic member provides a flexion moment when the patient is seated. As the patient arises, the flexion moment progressively diminishes and then converts to a progressively increasing extension moment as the patient continues to rise. The knee braces shown are expected to cause discomfort to a user when using the assistive device for a prolonged period and also, the assistive device shown is very likely to be noticed by other persons.

A knee joint assistant device using elasticity coefficient change and an optimized method of walking energy using thereof capable of including an elastic tool and control unit which controls the elasticity coefficient is known from KR 1020120062375A.

From EP 0205785 B1 a knee joint is known having a lower leg part articulated on a thigh part and arrested in the extended position of the knee, the arresting device of which is arranged in the lower leg part and projects into a recess of the thigh part with an interlocking element and extending upwardly beyond the contour of the lower leg part, the arresting being releasable by means of a Bowden drive introduced into the lower leg part via a rocker mounted in the lower leg part, the first arm of said rocker determining the position of the interlocking element and the second arm thereof being connected to the Bowden Drive, wherein an assembly block inserted into the lower leg part both guides and retains a pin serving as interlocking element which is urged into the arresting position by means of the spring retained in the assembly block and carries the rocker at its bottom side, the first arm of which is connected to the lower end of the pin. This arrestable knee joint is to be used by patients having a prosthesis to allow a secure feeling when walking with the prosthesis. This will serve to assist a natural limb during movement. It will be understood that the mechanical arrangement of the known device also is not restricted by anatomical aspects of a normal leg since it is to be used with a prosthesis which can easily be adapted.

From EP 0 564 734 A1, an adjustable orthosis is known for stretching tissue by moving a joint between first and second relatively pivotable body portions including a first arm with a cuff at its outer end for releasably attaching the first arm to the first body portion, and a second arm with a cuff at its outer end for releasably attaching the second arm to the second body portion. The document suggests that in therapy, one must first develop a range of motion before strengthening muscles and that the most difficult area is to work on the extremes of motion which should be done several times a day. The device neither can be considered unobtrusive nor is there any indication that it is assists motion or stabilizes stance or postures rather than only stretching tissue around a joint.

From US 6 635 024 B2, an articulating knee brace is known in which opposed lockable knee joint assemblies conjoin femoral and tibial links and are selectively lockable to inhibit relative rotation of the knee joint of the wearer when the wearer stands or otherwise applies weight to a foot and releasably to permit articulation of the knee joint when such weight is released. The known brace has components arranged at both sides of a leg that are lateral and medial of the knee joint. In particular in those cases where articulating knee supports are needed for both knees, a problem may occur in that the medial parts of the left and right braces touch each other, creating additional difficulties when walking, in particular for users that are weak, have particularly low control over their limbs or suffer from additional medical conditions such as imbalance.

From US 9 597 217 B2, a cable driven actuator is known that involves a movable link movable about a path by a cable connected to the link, and a movable support member having a cable routing element. The support member is movable in a manner to change a moment arm of the cable acting on a link to control torque applied to the joint. In order to allow a change of the moment arm, the device needs to have a rather large size. Also, the arrangement needs to be aligned rather precisely with the joint to be supported. Accordingly, even small misalignments of the orthosis caused by use might be displeasing for a patient.

From WO 2015/157731, an orthopedic device is known that includes rigid members for coupling two portions of a limb that includes a joint, and a cable of that couples to the rigid members and extends up to a powered element. The orthopedic device is configured to produce beneficial forces using the rigid member and the cable and beneficial forces are translated to the wearer. A control system generates control signals for controlling the powered element. The document states that while standard knee braces would generally support the knee in the body's frontal plane, they would not be helpful for supporting and buffering the knee against motion and the body's sagittal plane. The known device makes use of Bowden cables and large moment arms to generate forces around the spanned joint. As the transmission of force is solely reliant on the length of the moment arm due to the force transmitting mechanism that reduces the distances of two distal points on a circular path anterior to the spanned joint, where the center of the circular path is aligned with joint axis, such a design is particularly disadvantageous when worn under clothes due to the protruding members. Also, while a Bowden cable helps to prevent generation of disadvantageous force components since it relies on forces transmission on stiff sheaths, guiding a Bowden cable to a driving actuator such as DC motor may cause discomfort to a user. Furthermore, due to its reliance on the stiff sheaths, the length of a Bowden cable cannot easily be adjusted to different body heights and shapes, it tends to buckle, and may even resist movement in some cases , in particular where it is intended to hide the device below clothes and the users to engage in the range of normal activities involving walking around, sitting, lying and so forth.

From WO 2018 023 109 A1, a powered gait assistance system is known comprising a first arm and a second arm coupled together to allow relative pivoting between the first and second arms, wherein the first arm is configured to be coupled to an upper leg portion of a patient and the second arm is configured to be coupled to a lower leg portion of the patient such that relative pivoting between the first and second arms is about an axis proximate the patient's native knee pivot axis; a torque applicator coupled to the first and second arms and operable to apply torque between the first and second arms to urge a relative pivoting between the first and second arms; a torque sensor that measures magnitude of torque applied to the first and second arms, an angle sensor that measures a relative angular position between the first and second arm; a foot sensor that measures contact of a patient's foot with the ground surface and a controller programmed to determine what stage of the patient's gait cycle the patient's leg is in based on signals from one or more of the foot sensor, the angle sensor and the torque sensor and based on the determined stage of the patient's gait cycle, cause the torque applicator to apply assistive or resistive torque between the first and second arms to assist or resist the patient's volitional knee muscle output during the selected output stages of the patient's gait cycle. The known device uses a rigid frame that extends to the feet. As the arms are rather rigid, they should be properly aligned relative to the knee joint and also, the arrangement shown will not go unnoticed. As the relative pivoting between the first and second arms needs to be about an axis proximate the patient's native knee pivot axis, the correct orientation of the entire device must be maintained by sufficiently strong braces or the like, reducing comfort to the user.

From WO 2018/122106 A1, a soft wearable muscle assisting device is known. It is stated that the known muscle assisting device can be used to assist in extending a leg. It is suggested to use a tendon that is unreeled or reeled as necessary. It is suggested to guide the tendon in two parts along the knee, namely a first part along the left side of the knee joint and the other tendon part along the right side of the knee. This can also be done for the elbow. It is also suggested to provide stretching bands in front of the knee and behind the hip. While the known device is a soft wearable device, a tension mechanism is suggested where the cables shall glide in a predetermined distance in front of a knee. Accordingly, as guiding the tendon in front of the knee usually necessitates to keep some distance from the knee, this also enlarges the system. Hence, it might be preferred in certain instances to have a less obtrusive wearable assistive device, in particular a less obtrusive active wearable assistive device.

Further wearable assistive devices are known from US 2010/056970 A1, US 2006/206043 A1 and US 2018/000623 A1.

US 2010/056970 A1 discloses a knee orthosis device with a pair of opposed first and second vertical struts connected to each other via a pair of oppositely arranged hinge elements. The knee orthosis device provides medial, lateral, and rotational control of a knee joint.

US 2006/206043 A1 discloses an articulating joint that is disengageable between two modes of operation, a unidirectional movement of the joint or bi-directional movement of the joint, with features that allow the mode to be automatically switched as required. When fully locked, the joint inhibits joint flexion while allowing joint extension. When actuated, a locking mechanism is disengaged to allow both uninhibited flexion and extension of the joint.

US 2018/000623 A1 discloses an orthopedic device including a hinge assembly, a frame having first and second frame components spaced apart from and connected to one another by the hinge assembly, a strap system extending between medial and lateral sides of the first frame component, and at least one tensioning element.

It is an object of the present invention to provide an improved wearable assistive device which overcomes the disadvantages of known devices.

This object is achieved by the features of what is claimed in an independent manner.

Some preferred embodiments can be found in the dependent claims. Further preferred embodiments can be found in the description hereinafter.

Accordingly, the present invention suggests a wearable assistive device comprising two, namely first and second, parts, wearable proximal and distal of a limb joint respectively and having an articulated interconnection and a tendon interconnection, guided from the first part to the second part and adapted to exert an assistive force on the limb joint when tensioned; wherein the articulated interconnection is provided unilaterally, preferably on the lateral side of the limb joint, the tendon interconnection is provided unilaterally, preferably on the lateral side of the limb joint, and counter-torsion means adapted to generate forces counteracting torsion of the first and second wearable parts on tensioning the tendon interconnection are provided. Preferably, the forces generated by the counter-torsion means counteract torsion of the first and second wearable parts relative to the limb on tensioning the tendon interconnection.

In other words, the present invention suggests that an articulated interconnection is used with a tendon and that both the articulated interconnection and the tendon interconnection are unilateral, thus reducing inter alia a danger that the interconnection means of the left leg interferes with those of the right leg.

Furthermore, even where a user needs active assistance for only one leg, an advantage is obtained over a brace arrangement interconnected at both (lateral and medial) sides as the device of the present invention can be made sufficiently unobtrusive to be worn below normal clothing.

Also, long-term use for a plurality of hours or an entire day becomes possible as the counter-torsion means of the present invention generate forces counteracting torsion of the wearable parts that would otherwise occur when the tendon interconnection is tensioned. This allows to reduce the forces necessary to attach the device.

In more detail, it has been realized that where a unilateral articulated interconnection is actuated by a unilateral tendon, torsion of the wearable parts may easily occur as the wearable parts will exert some force on the limb. If this is done on one side only, some form of twisting and relative torsion of the wearable parts must be expected. This holds in particular where the tendon is not actuated directly on the limb, for example by a motor attached directly to the limb, but is attached further away from the limb and this spans multiple joints, for example at the torso. Thus, in such a situation, use of a Bowden cable relying on force transmission through a stiff sheath is to be avoided, given the disadvantages mentioned above. It will be understood that for a motor directly attached, a Bowden cable also does not offer any advantage, so that use of a Bowden cable instead of a tendon interconnection is to be avoided as well. This also holds where the two wearable parts do not comprise two rigid parts that completely encircle the limb. Note that such an arrangement would be bothersome to wear. Typically, the wearable proximal and distal parts of the invention will comprise soft components providing some tolerance needed to prevent undue pressure on the limb.

Accordingly, when tensioning the tendon of an arrangement having at least one and preferably two parts wearable proximal and distal of the limb joint, respectively, and having at least one soft component for attaching to the limb, torsion on tensioning a unilateral tendon interconnection will occur, previously reducing user comfort. However, by now generating forces counteracting such twisting and/or torsion, comfort to a user can be increased and it is no longer necessary to exert a high pressure on the limb and/or shear forces merely to fix the parts even during a phase when no assistance is needed.

Therefore, the counter torsion arrangement of the present invention increases comfort to a user of an active wearable assistive device, while increasing the stability of the system at the same time.

Preferably, the tendon will be actuated by an electric motor, in particular a DC motor as it has been found that DC motors can provide sufficient forces, supply sufficient power and have a good temporal response.

It will be understood that the wearable assistive device while comprising several parts explicitly mentioned in the independent claim may also comprise additional parts such as a motor, a control, a sensor, batteries and so forth. It is also noted that the wearable assistive device may assist not only one single limb joint or the same joint on both a right and a left limb such as the left and right knee. Rather, assistance may be provided to a number of joints.

Each joint may be assisted separately. Alternatively, assistance may be provided to several joints by one and the same tendon. In specific cases, a situation can be considered, where even the left or the right side are provided assistance together by the same motor and/or tendon.

For example, it would be possible to design a wearable assistive device that has the two parts having an articulated interconnection and tendon interconnection with the articulated interconnection and the tendon interconnection each being provided unilaterally and also being provided with a counter torsion means to assist a knee, but in a manner such that where the tendon is tensioned, assistance is also provided to the hip of the same leg and/or a foot.

Accordingly, the claims should not be construed to imply that a wearable assistive device only comprises those parts explicitly mentioned.

Preferably, a motor adapted to tension the tendon interconnection can be provided at the torso and the tendon is then guided from the torso to a first of the two parts. Arranging the motor close to the torso is well known and has been used with a plurality of wearable assistive devices designed and offered by the applicant. From this, methods to guide the tendon interconnection from the motor to a first of the two parts along the body are known, in particular using a soft flexible sheath that is not used to transmit collinear forces like a Bowden cable. It will be understood that in a preferred embodiment, the tendon can be guided across the hip to the knee so as to provide assistance to the knee.

Preferably, the tensioning of the device is controlled by a control in a manner stabilizing the posture and/or in a manner supporting an extension of the limb, and particular an extension of the knee. The control may comprise a microcontroller executing a control program and processing input related to sensor signals, for example inertial measurement units (IMUs), that are integrated in the rigid plates, detecting different accelerations and angular velocities, combined with filtered angles from these signals indicating whether or not a person currently has lifted a foot or is standing firmly on the foot or is performing activities of the daily living such as walking, stair climbing, or sitting .

Furthermore and/or as an alternative, one or more foot pressure sensors, load cells, electromyography sensors, ultrasonic sensors, strain gauges, or other means of angle measurements like encoders alone or in combination with IMUs can be attached to a limb or several parts of the limb to be assisted and/or to the corresponding other limb, even if such other limb currently does not need support. Sensor signals from two limbs, even where only one limb is to be supported, can be detected and evaluated as this helps to detect a gait phase and/or a current posture-or a change in a current posture. Detecting a gait phase, gait characteristics, a posture and/or changes in a posture is well known in the art. However, while the wearable active assistive device of the present invention is in a particularly preferred embodiment used to stabilize the knee in a given posture, in particular while standing (which may be difficult for person suffering from certain medical conditions) and/or in a manner supporting an extension of the knee, the variable assistive device of the present invention could also be used to extend for example an elbow or the shoulder and/or to flex or straighten the ankle.

Preferably, at least one and preferably both of the two parts wearable proximal and distal of the limb joint comprise a solid plate adapted to be nestled in use to the limb as well as a strap arrangement comprising one or more straps for attaching the plate on the limb. Preferably, the plates will be padded. Due to its structure and the generated rotating points, padding on the proximal and distal parts close to the spanned joint, is particularly useful to increase user comfort. Because the plate acts as moment arm aligned with the user's limbs to prevent the rotation around the aforementioned rotation points, the plates should have a sufficient length, preferably only a part thereof is padded as only specific parts of the plates have to be in contact with the user and require padding which improves the breathability of the entire structure.

By using a combination of a solid plate and a strap arrangement, it is possible to provide for a sufficient stability of the articulated interconnection while maintaining a high comfort to the user by a softer strap arrangement. Additionally, the configuration allows for reliable force transmission even with a slightly shifted or shifting center of rotation that is not perfectly aligned with the joint or does not stay perfectly aligned with the joints.

Preferably, the strap arrangement is adapted for attaching the plate on the limb such that at least one strap will extend from one side of the plate around a posterior part of the limb and to an opposite side of the plate. Accordingly, the plate will be worn anterior while the straps are provided posterior. This is particularly advantageous at the shank, where the solid plate is close to the shin and hence is affected less by the activity of sural muscles. The straps can easily accommodate to the changing shape of these muscles if used, so comfort will be high. This is preferably achieved by routing the upper strap above the gastrocnemius muscle and the lower strap below the muscle insertion, right above where the achilles tendon is typically visible. Similarly, providing the solid plate at the front of the thigh is preferred so as to increase comfort to the user as a user frequently is more sensitive at the inner (medial) parts of the thigh and/or the back of the thigh where the softer straps can be arranged.

Preferably, the strap arrangement is adapted for attaching the plate on the limb such that at least one strap is self-tensioning. Tensioning the strap in use counteracts twisting or torsion and hence a self-tensioning strap or self-tensioning strap arrangement implements one embodiment of a counter-torsion means. Preferably, a plurality of straps is used, in particular two straps for each plate. Using several separate straps prevents discomfort due to wrinkles, reduces movement, prevents discomfort due to sweating and so forth.

Where several, in particular four straps are used for the two parts, a self-tensioning is preferably implemented at least for one strap, in particular the most proximal strap. This holds in particular for the most proximal thigh strap.

Preferably, the at least one self-tensioning strap is adapted to be guided from one side of the plate, around a posterior part of the limb, to an opposite side of the plate, and to the tendon interconnection. Preferably, the tension generated within the self-tensioning strap upon tensioning the tendon interconnection, during use, generates a counter-torsional moment acting on the limb via the solid plate.

Regarding tensioning of a strap in use it is noted that self-tensioning arrangements have been described in WO 2018/122106 which is enclosed herewith for reference in its entirety, in particular for reference to self-tensioning. Note that in this known prior art, reference is also made to self-tightening mechanisms. It is noted in particular that it has been stated that certain structures can be provided that automatically tighten when forces are applied, for example where a criss-cross or zig-zag structure is provided. Also, active tendons are suggested to be directly connected to structures as necessary. It is also stated that stiffness can be increased by forming a rigid bond, for example by sheets having low adhesion in one flexibility when not compressed and high friction coefficients and thus rigidity when compressed. The required compression forces have been disclosed to be achievable through the structure itself, pneumatically and/or through electro-adhesion allowing a base layer to take on a body shape curing actuation. It has been stated that an increase of the contact area and the human body and increasing and optimizing the contact area will result in low normal and shear stress. The increased stiffness has been disclosed to reduce the compression forces through a circular structure that distributes the forces within its structure when rigid; note that in a similar manner, even where no full circular structure is used, but a combination of a plate and corresponding self tightening straps, the same advantage as disclosed in the prior art by the applicant can be obtained.

Preferably, a tendon of the tendon interconnection is guided through a guiding member having flexibility higher than that of the solid plate but lower than that of the tendon and to attach the guiding member fixedly to a strap, in particular a thigh strap, in particular the proximal thigh strap. Such guiding member will also contribute and, in certain cases even suffice as a counter torsion means.

Preferably, the joint to be assisted is the knee and one of the plates is adapted to be positioned in use on the proximal anterior and lateral sides of the shank, in particular such that in use the anterior part of the shank plate will cover an area around the tibial tubercle. It will be understood that terms such as "around the tibial tubercle" are to be construed in a manner meaning that a plate resides on the user's limb close to the tibial tubercle and extending along, but not necessarily encircling the tibial tubercle. It should be noted that typically a few millimeters or even up to 1cm- 2 cm of a distance between the shank plate and the tibial tubercle will not be critical. Note that typically, the edge of the shank plate will be positioned accordingly so as to be close to the tibial tubercle.

In similar manner, for a knee assisting wearable active device, where one of the plates is adapted to be worn at distal anterior and lateral sides of the thigh, in a preferred embodiment, the corresponding plate will be worn such that in use the thigh plate rests on the anterior part of the quadriceps muscle, in particular just above the quadriceps tendon.

The plates may have a base part made of plastic material as this is lightweight, hygienic, cheap, and will not cause irritation of the skin. It will be understood by a skilled person that a variety of plastic materials can be used such as carbon fiber, carbon fiber reinforced resin, ABS, Teflon, polyamide, as well as polyamide that is carbon fiber or glass bead reinforced Polycarbonate. In a practical embodiment, a PA12-plastic material has been used to produce the plates in a multi-jet fusion process. The E-module of the plate in this practical embodiment is 2MPa, the tensile strength is 49MPa, the elongation of break is 20%.

Typically, it is preferred to allow for some elongation before breaking so as to avoid destruction of the plate during cleaning, handling, attaching and/or use. Allowing for some elongation helps to accommodate different limb shapes and/or sizes, which is particularly helpful for the thigh. Having a plate that allows accommodation to different sizes and/or shapes allows to use ready-made plates rather than having to customize the plates for every patient. Note that a padding can be used to adapt a ready-made plate to a patient in an easy manner. In the same manner, the element should not be too rigid nor should it be so soft because in both cases, a transfer of forces may be impaired. The average skilled person will understand that the parameters indicated for the material of the practical embodiment may guide him to possible sets of parameters, but are not intended to be limiting at all. It is easily possible to have variations of the indicated of -50% to +100% or more. It will be understood that also, the geometric properties and topology of the plate will have an impact of durability, rigidity and so forth.

In case the wearable assistive device is used as an embodiment wherein the articulated interconnection is provided at the lateral side of the knee joint, it is preferred that a hinge joint having a center of rotation aligned at least approximately with that of the knee joint is used. The alignment of the hinge joint does not have to be perfect. It is sufficient if an approximate alignment is achieved, in particular to only such a degree that any deviation can still be compensated in use by the compression of the device and/or the compression of the tissue of the user.

Note that while the two parts need to be articulated relative to one another, it is not vital to have a hinge joint having a single, well-defined axis of rotation. While typically the axis is well defined, for example because a hollow cylinder is provided to house a pivot with a clearance very small, it is possible to have a hole or through hole somewhat larger than the pivot so that a larger clearance is obtained. Furthermore, a series of interconnected hinges might be used. However, using a single hinge joint having but a small clearance and a sufficiently low friction is preferred.

It is noted that the parts of the hinge joint can be made integral with the respective plates, in particular where a base part made of plastic material is used, but that also, separate parts could be used, in particular so as to have a more robust bearing of the hinge joint and/or to achieve a lower friction.

Also, because a situation may occur where a user applies lateral forces to the device, in particular when cleaning or handling the wearable assistive device and/or the plates, it might be preferred to have a particular sturdy joint. Accordingly, use of a material other than that of the plates might be preferable.

Preferably, the hinge joint is also adapted to guide the tendon and it will extend anteriorly from the thigh or shank half of the joint axis when viewed in the sagittal plane.

In this context, it should be understood that using a tendon that is attached on one of the plates, guided across the joint and reeled up on the other side will exert a force on the joint to be assisted. In particular, it is important to realize that when changing the angle of the joint to be assisted changes the path and hence the length of the tendon changes. Vice versa, if the length of the tendon changes, the angle of the joint follows correspondingly. Accordingly, shortening the tendon is possible if the joint angle changes and hence, a force is exerted. This holds even in case the tendon is not running within a plane. Accordingly, it is possible to first route the tendon in a plane perpendicular to the joint axis but to then guide the tendon away from this plane, in particular away from the lateral hinge joint towards a position closer towards an anterior central point of the proximal plate. In this manner, where a tendon starts collinear to the lateral edge of the knee joint and generally perpendicular to the axis of the hinge joint, an angle β to the collinear path is obtained.

In this manner, a force component is exerted on the lower plate relative to the upper plate where the tendon is guided by guiding means provided on the proximal plate.

More generally speaking it is preferred that each of the first and second parts comprises a solid plate adapted to be nestled in use to the limb, wherein routing means are provided to route the tendon interconnection from a hinge joint between the two plates towards an anterior central point of the proximal plate. Preferably, the tendon interconnection is adapted to be routed such that the tendon is guided laterally from a distal position below, e.g., the knee joint (or another joint) to a more medial position proximal of, e.g., the knee joint (or another joint), in particular at an angle β between 5° and 45°, preferably between 5° and 25°. Accordingly, this counteracts torsion and implements another embodiment of counter torsion means.

Preferably, a plurality of guiding means is provided at a plate as to allow variation of the angle β. This in turn allows to adjust the counter torsion means to the particular anatomy of a given patient. In a preferred embodiment, an angle β at least between 5 to 25°, preferably between 1 to 30° can be selected.

Preferably, a variation of the angle β is possible, wherein preferably at least two different angles (other than a collinear arrangement) can be selected, preferably at least three or four different angles.

The present invention will now be disclosed with reference to the drawings by way of non-limiting examples. In the drawings,

Fig. 1 shows a leg to which the wearable assistive device is attached in a front view, a side view and a back view;
Fig. 2 shows the lower plate without tendon and without straps;
Fig. 3 shows a thigh plate used in figure 1;
Fig. 4 shows a simplified scheme of the human leg with one degree of freedom;
Fig. 5 shows the model usable in a virtual work analysis;
Fig. 6 shows the relation between a knee angle and the knee moment;
Fig. 7 shows the relation between a hip moment and the knee angle;
Fig. 8 explains a preferred detail of a wearable assisting device wherein a spring as resilient elastic element provided in series with a tendon which in turn is fixed to a plate attached to a limb;
Fig. 9 shows a frontal plane view of a thigh brace of a wearable device according to a preferred embodiment of the present invention in order to display the transverse, sagittal and frontal planes;
Fig. 10 schematically shows the moments generated within the transverse plane without self-tensioning strap; and
Fig. 11 schematically shows the moments generated within the transverse plane with a preferred embodiment of a self-tensioning strap.

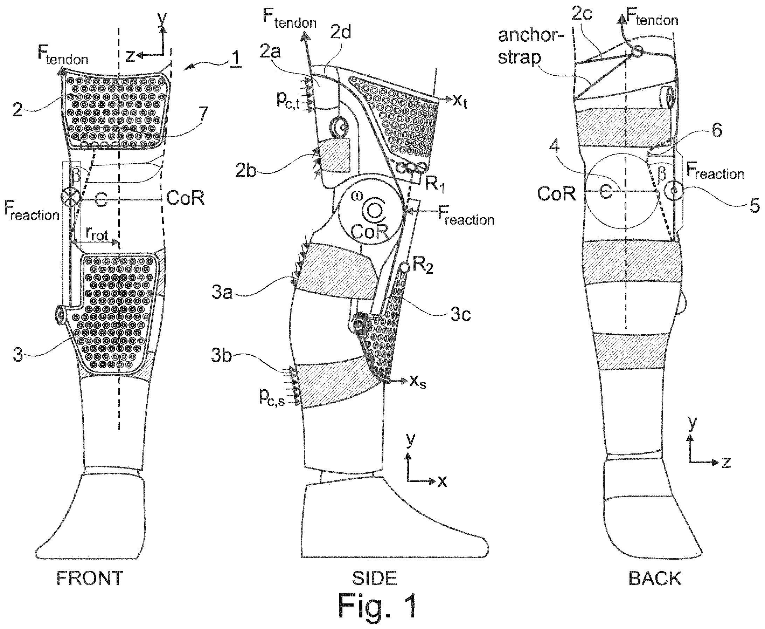

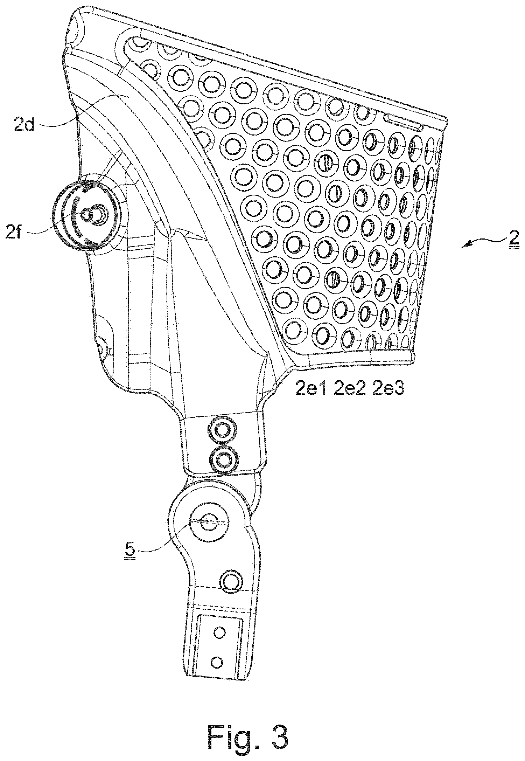

According to Fig. 1, a wearable assistive device 1 comprises two parts 2, 3 wearable proximal (cf. part 2) and distal (cf. part 3) of a limb joint 4, respectively, and having an articulated interconnection 5 (cf. also fig. 3) and a tendon interconnection 6 guided from the first part to the second part and exerting an assistive force $F_{reaction}$ on the limb joint 4 when tensioned as indicated by $F_{tendon}$, wherein the articulated interconnection 5 is provided unilaterally, the tendon interconnection 6 is provided unilaterally and counter torsion means 7 adapted to generate forces counteracting torsion of the wearable parts 2, 3 on tensioning the tendon interconnection are provided.

In the embodiment shown in Fig. 1, the wearable assistive device is a leg assistive device and the limb joint 4 is a knee. The wearable assistive device 1 is an active wearable assistive device wherein the tendon of the tendon interconnection 5 is actuated by a DC motor provided at a breast or back plate on the torso of a user (not shown). The tendon is routed across the hip to the motor.

The two parts wearable proximal and distal of the knee comprise plates 2 and 3, each plate being attachable to the leg with 2 straps 2a, 2b and 3a, 3b, respectively.

As can be estimated from the three views shown in Fig. 1, the components of the wearable assistive device attached to the leg do not extend far away from the leg and therefore, these parts of the wearable assistive device can be worn underneath a trouser or the like. Accordingly, the wearable assistive device is unobtrusive.

Thigh plate 2 as shown in Fig. 3 has a front part having a plurality of holes to reduce transpiration and increase comfort to the user. Thigh plate 2 is shaped and sized to be worn anterior with straps extending posterior from the left side of the plate to the right side at two positions, namely a proximal and a more distal position. Note that the thigh part provides a stiff pivot support, designated as (R1) and that the calf part also provides stiff pivot support designated as (R2).

It is noted that a large distance between the two straps attached to plate 2 (and, similarly a large distance between the two straps 3a, 3b attached to plate 3) helps to prevent rotation or other movement of the plate relative to the leg when exerting forces by tensioning the tendon interconnection. The straps 2a, 2b are flexible enough to be comfortable, but are tight enough to still keep the plates in their position without major movements. It will be understood that plate 2 - and, similarly, plate 3, will be padded to increase the comfort to the user. The padding may allow diffusion to increase breathability; also, it is preferred to not cover the entire inner area of the plate with padding but to leave one or more cutouts. In particular, the padding can have a large central opening. Similar padding is provided for plate 3. Also note that in a lightweight arrangement, plates 2 and 3 may have a central cutout as well.

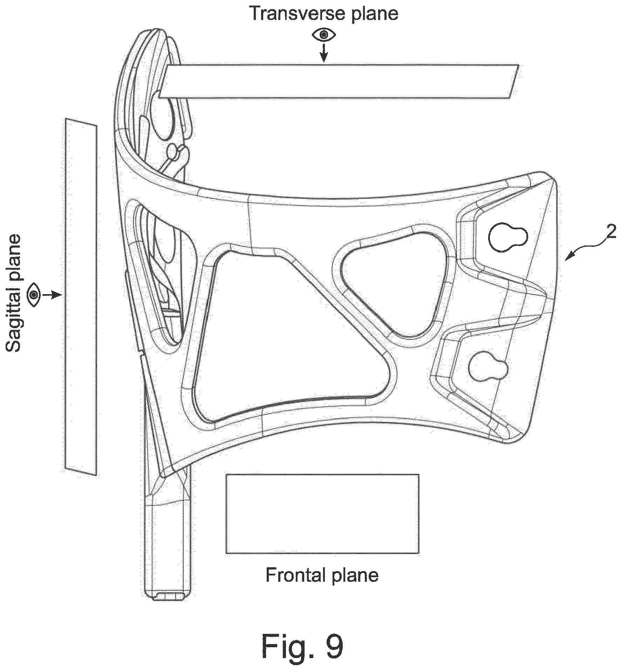

As can be seen in Fig. 1, (cf. the back view), the upper strap 2a, that is the most proximal strap on the thigh, has an anchor strap 2c through which the tendon is guided in a manner such that, when the tendon is tensioned, the strap is pressed against the skin of a user to a higher degree. Accordingly, the strap is self-tensioning and the self-tensioning of the strap counteracts twisting or torsion in use so that the anchor strap 2c can be considered a counter torsion means. A counter-torsion means based on such self-tensioning will be explained in more detail further below with reference to Figs. 9-11.

It is noted that the straps will have some flexibility and that they preferably can be replaced easily. Accordingly, a Velcro-like attachment to the plate is preferred on at least one side of the plate.

Tendon 6 is guided in a trench-like structure 2d along the edge of the plate. Furthermore, the through-holes on the front side of the plate not only serve to increase breathability, but may also be used to insert studs, screws or other structures that allow to guide the tendon sideways towards the center of the leg, that is closer to the knee cap. Through-holes suitable for this are indicated as 2e1, 2e2, 2e3.

As will be seen in Fig. 1, tendon 6 is routed in a manner that leads to an angle $\beta$ of the tendon relative to a plane comprising the joint 5 and the lateral edge of the knee cap and the lateral edge of the knee cap. The angle $\beta$ helps to reduce any tendency of relative twist or torsion between upper and lower plate on tensioning the tendon and hence serves as a counter torsion means. This can be done in a manner selecting a through-hole 2e1 or 2e2 or 2e3 suitable for a given user to insert a tendon deflecting stud.

Whereas plate 2 is made of plastics material, for example in a practical embodiment made of polyamide, the joint 5 made of metal, produced separately and attached to the plate 2 with screws or the like. Note that plate 3 is also made of plastic material.

It will be understood that the plate 2 has a thickness and shape allowing some deformation so that a ready-made plate can be used without customization for every user. The same holds, obviously, for plate 3.

It is noted that the straps could be tensioned by suitable tensioning mechanisms such as shown by reference numeral 2f showing a tensioning mechanism commonly known in the art; however, such tensioning mechanism is not deemed vital.

The joint 5 is arranged such that for an average user, the axis of joint 5 is collinear with an axis of a knee joint movement. Note that given the anatomical shape of the thigh (having a large circumference closer to the hip compared to the circumference closer to the knee), and given the anatomical form of the calf, the parts of the joint 5 will not have to extend laterally beyond a general contour tangent to the outermost parts of the thigh and the calf, respectively. Accordingly, the wearable assistive device can be worn below underneath normal clothing, in particular in view of the fact that soft tendons 6 requiring no stiff sheaths can be used.

It will be understood that tendons having a minimum radius of curvature below 3 cm are highly preferred. A preferred embodiment may use Dyneema tendons.

Regarding plate 3, straps 3a and 3b are arranged such that the upper strap is routed above the gastrocnemius muscle and the lower strap is routed below the muscle insertion, right above where the Achilles tendon typically is visible. It is noted that the anatomical form of the calf helps to position the lower plate using such a strap arrangement and to keep the lower plate correctly positioned.

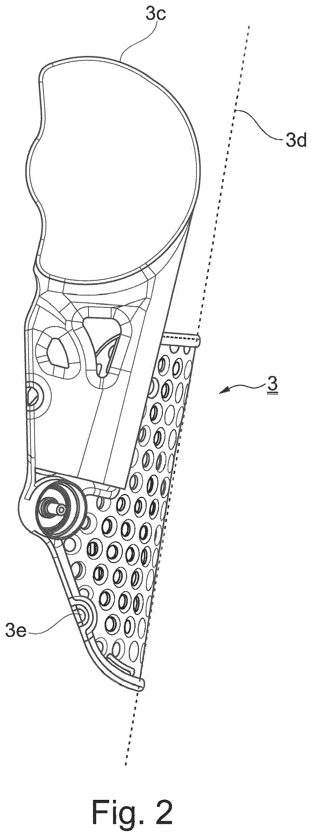

Furthermore, as can be seen in Fig. 2, shank plate 3 comprises a tendon guide element 3c which in use extends anterior of joint axis 5. However, guide 3c does not need extend beyond the front of lower plate 3divided as indicated by dashed line 3d.

Note that guide 3c is integral with the guide plate and thus built from plastic material. The same holds for a hinge cover that covers hinge 5 such that during movement of the hinge, no textile material from clothing worn above the wearable assistive device can be pinched or jam the movement of the joint. The tendon is guided on the outer perimeter of the cover, which however does not extend anterior of the leg. From the analysis below, it will be understood that sufficient support can be provided to the leg despite this small dimension.

For anchoring the tendon, at the lower part of the plate 3, a through-hole 3e is provided, through which the tendon can be guided and to which the tendon can be fixed using a knot.

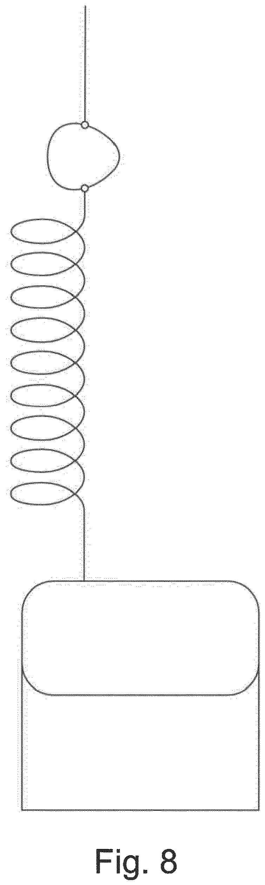

In a preferred embodiment, it is possible to provide a resilient elastic element in series with a tendon and to then fix the resilient elastic element to the through-hole 3e. This will be described below with reference to Fig. 8. In more detail, it is possible to implement a preferred embodiment that has a transparency mode and/or provides other advantages. Note that Fig. 8 is derived from DE 10 2018 215 163.6. In DE 10 2018 215 163.6, a wearable active assisting device has been suggested having a selectable minimum degree of assistance, which can be close to zero limb assistance, by actuating the motors usually assisting the limb in a manner so that the elongation of the force transmission element is tightly controlled according to a model derived based on the plurality of sensor signals. This allows selecting minimum assistance without decoupling the actuators, motors and the like from of the tendons. In particular, it is possible that the minimum degree of limb assistance is selected not by the user wearing the wearable active assisting device himself but by a physical therapist, physiotherapist, medical doctor and so forth, in particular even without the patient noticing. As the motor continues to elongate or shorten at least one force transmission element, even where no actual support is provided, a user hearing the motor will have the impression of being supported. Thus, a placebo effect of the wearable active assisting device can easily be tested, in particular where a patient has to rebuild confidence in his (or her) own muscles. What is more is that by modeling elongation and by shortening and/or elongating the at least one force transmission element, in case where it turns out that assistance must still be provided for certain movements in spite of the expectation of the physiotherapist or the like, assistance can and will immediately be available. It should be noted that for the purpose of the present invention, a limb assistance degree selection input is adapted so that with the unit switched on at least 2 different degrees of support are selectable, the minimum degree of limb assistance being one of these degrees selectable even where this minimum degree corresponds to zero assistance.

It has also been suggested in the document previously discussed, that it is highly preferred to allow that the wearable active assisting device can be used without determining dedicated physical parameters for each single user overly exact. The same holds for the present invention. In a highly preferred embodiment disclosed in the reference cited herein, between the limb and a motor, a resilient element is provided in series with the force transmission element to be elongated or shortened. Using such a resilient element, for example a coil spring, allows small errors in the determination of an elongation or shortening of the force transmission element currently needed to remain undetectable to a user. This can be done in the present application as well. Accordingly, in a preferred embodiment of the present invention, it is preferred that a restrictor is provided that limits or restricts the elongation of the resilient element, for example restricts the elongation of the spring to a maximum allowed elongation, and that takes up any additional forces else applied to the spring or other resilient element without allowing further elongation thereof. For example, a specific length of a cord or wire could be provided within a coil spring. The cord could be attached at the same points as the ends of the spring, so that the restrictor also would be placed between the limb and a motor used for assisting the limb when actuated. Given that the rope shall be longer than the spring coil as long as the spring coil is not extended, all forces exerted on the force transmission element, for example due to the mismatch between model and user, will lead to an extension of the spring to a certain degree. A corresponding example is shown in Fig. 8. Note that in Fig. 8, a cuff drawn as a closed ring like structure is shown that would be attached to a limb, but that in a practical embodiment, the cuff would of course be replaced by the plate/straps-arrangement shown in Fig. 1.

In the arrangement shown in Fig. 8, while the extension of the spring (shown as a coil spring) remains low, no forces are taken up by the restrictor shown as a central wire running within the coil. However, once the spring is extended to an allowed maximum, any additional forces will be taken up by the restrictor and will thus not allow further extension of the resilient spring. In other words, the restrictor restricts elongation to a specific maximum allowed in particular where actual support or assistance is provided. By properly selecting an adequate maximum allowed elongation of the resilient spring element and a suitable modulus of resilience, care can be taken that any deviation between the model and an extension actually needed for a specific user will neither impair the intended behavior of the present wearable active assisting device during actual assistance nor will it render the device sensible during a transparency mode.

In a preferred embodiment, the resilient element has a modulus of resilience such that for a maximum residual force acceptable in a selected minimum degree of limb assistance, the resilient element is elongated by no more than the maximum allowed deviation between a standardized model and the correct extension for a given user.

In this context, it will be obvious that despite not having to take very precise measures of a user, it is possible and will be preferred to provide a plurality of resilient elements differing both in resilience and maximum allowed length. In a typical situation, the maximum allowed deviation may be a few centimeters, for example 1 to 7 cm. This distance can easily be overcome even in case of an emergency change of limb assistance given standard motor speeds. These maximum allowable lengths that are preferred in turn allow residual forces due to the extension of the resilient element when mismatched to the model that are hardly detectable by a user and will ensure that a mismatch remains undetected most or all of the time.

As indicated above, it is possible to determine a phase in current movement identified and to output a motor actuation signal in response to a modeled elongation. While it would be possible to predict the actual extension, however, it should be noted that for the transparency mode provided by a wearable active assisting device according to the present invention, neither knowledge of for example a gait phase is needed nor does the system rely on an accurate gait cycle, since the actuation profile is not predefined. In particular, it should be noted that instead of relying on specific gait phases, other parameters such as a continuous force scaling depending on the knee angle asf. can be used.

However, nonetheless, the control may be adapted to identify certain activities such as walking, standing, walking uphill or downhill, ascending or descending a stair and so forth. As indicated above, even where the control of the transparency mode itself need not rely on the determination of the precise current activity, safety of the device can be increased.

Accordingly, it can be seen that in a preferred embodiment, it is desirable to use the resilient element as explained with respect to Fig. 8 so as to use general parameters and to elongate or shorten the tendon only to a precision such that the spring is not fully extended during a mode so that a transparency mode can be implemented. Only when actual assistance is needed, for example because a patient becomes exhausted, the tendon will be shortened so much that the element central within the spring coil is no longer slack. As the distance the tendon has to be reeled in will be extremely small during such a transparency mode, active assistance can be provided almost immediately and without causing a shock or jerk to the limb supported.

It should thus be noted that one possible embodiment can be a wearable active assisting device comprising an actuator in use to provide a limb assistance and coupled to a limb to be actively assisted via at least one force transmission element to be elongated or shortened by the actuator; and a control having an input for signals from a plurality of sensors, a signal processing stage for processing input signals from the plurality of sensors, and an output stage for outputting a motor actuation signal in accordance with the processed sensor signals; wherein the control further has a limb assistance degree selection input for selecting a degree of limb assistance; and wherein the signal processor stage is adapted to continuously model an elongation of the at least one force transmission element to be elongated or shortened corresponding to a movement or posture currently detected by the plurality of sensors to output a continuous actuator actuation signal according to a modeled elongation of the at least one force transmission element to be elongated or shortened and in response to a selected minimum degree of limb assistance. In other words, the device of the preferred embodiment can be used in a transparency mode.

It will be understood that the wearable assistive device of the present invention is an active wearable assistive device having one or more motors, a control and several sensors that allow the control to energize the motor as necessary to provide assistance to the user.

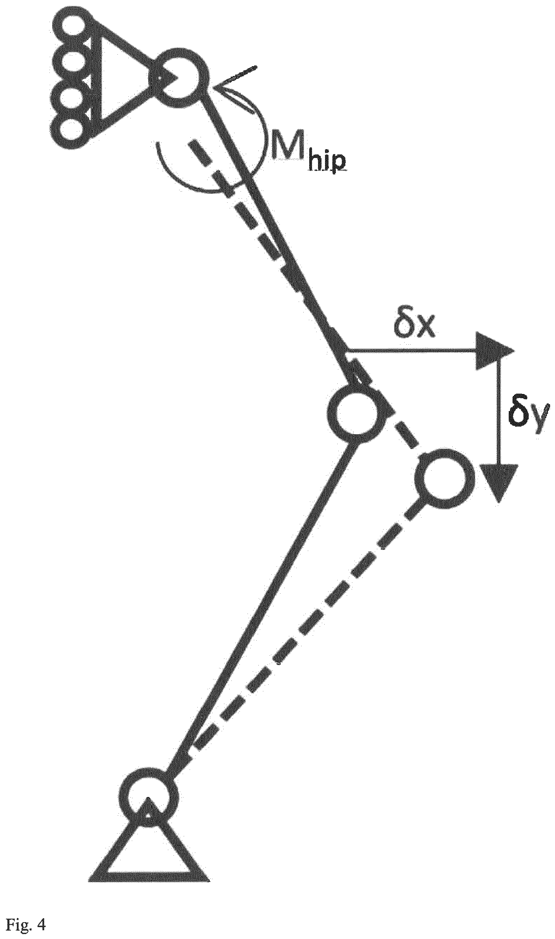

The motor(s), control and sensors are not shown, as the highly flexible tendon allows to use a known arrangement attachable e.g. to the torso of a user. However, the control must be adapted to provide a required level of assistance. To this end, it has already been generally suggested that active wearable assistive device model a necessary elongation or shortening of a tendon in view of a current posture, gait phase and an expected or detected movement. Once the model has predicted a necessary elongation or shortening of a tendon, it is possible for the control to energize the motor correspondingly. It will be understood that for the active wearable assistive device, a control can be implemented in this manner. To this end, a possible model is disclosed hereinafter with respect to Fig. 4-7.

With respect to this model, the following is noted. Previously, the significance of the Knee Moment Arm (KMA) has been attributed to the moment produced around the knee joint. In such previous model, the moment around hip and ankle joints were ignored. Also, previously, a swing double pendulum model was used for the system. In such case, the moment generated around the knee is a crucial component of the knee extension.

However, the present inventions has realized that the situation in a stance phase changes the arrangement of the double pendulum system, locking the ankle and hip joints. The extension of the knee joint thus becomes a result of assistive moments generated around the hip and ankle joints.

For the model, it is assumed that assistance of the active part of the wearable assistive device is provided during the stance phase only. It has been found that in such a case, the size of the KMA is no longer that critical to the moments generated around the hip and ankle joints in view of the following analysis.

For the purpose of the analysis, the human body was simplified to a 1DOF frame as shown in Fig. 4.

In Fig. 4, A is the ankle joint (X-Y supported), H is the hip joint (X-supported) and K is the knee joint. KF is the knee moment arm. Thip and Tshank are the tendons above and below the Knee-Moment-Arm KMA, respectively, x and y subscripts are for x and y components of the respective tendons.

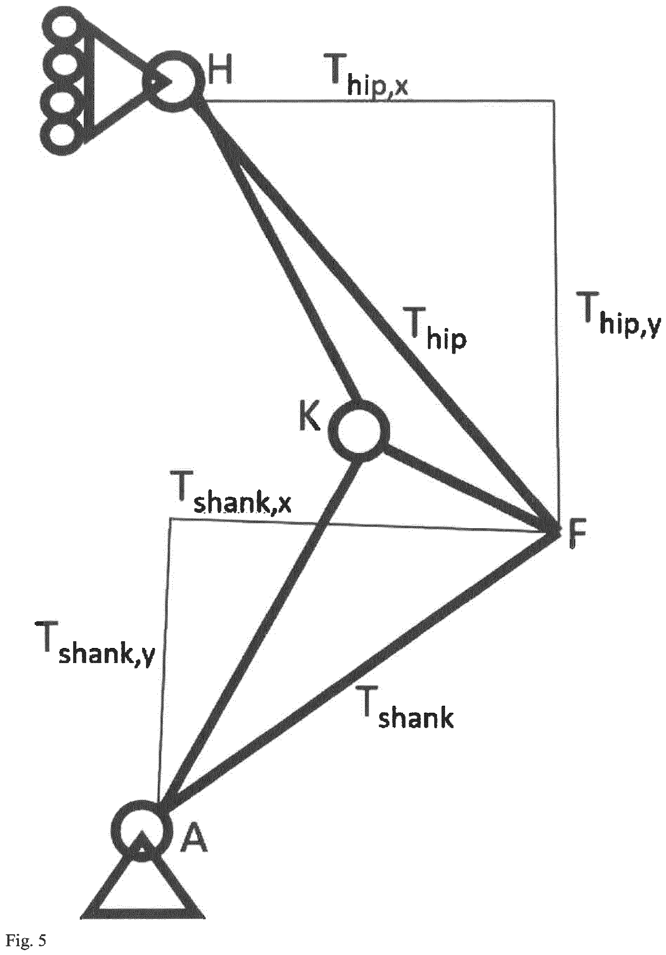

Using this terminology, a virtual work method can be used for an analysis. In more detail, the virtual work model shown in Fig. 5 was used.

Using this approach, the moment that needs to be generated in order to resist the kinematic change in the H system induced by the sum of hip and thigh tendon forces around K can be determined.

Consider that dx and dy show the migration of K due to an infinitely small deformation in the system corresponding to work done by Mhip. Mhip will then perform work equal to:

$$U = Mhip * d(aHKA),$$

where aHKA is the knee angle and d(aHKA) is the change in the knee angle. Other forces that would perform external work on the system would be the vertical and horizontal projections of the tendon forces around the K:

$$(Tshank,x + Thip,x) * dx \text{ and } (Tshank,y + Thip,y) * dy$$

Accordingly, the total virtual work equation can be shown to become:

$$0 = -M_{hip}*\delta(aHKA) + (T_{shank,y} - T_{hip,y})*\delta(y) + (T_{shank,x} + T_{thigh,x})*\delta(x)$$

Note that the negative sign before hip moment is because the work done by that moment is negative; in other words, it is putting work into the system. The negative sign of the vertical hip force is due to the negative direction of the force.

Having established the total work equation, the d(y) and d(x) parts of the equation can be expressed using the knee angle (d(aHKA)) and accordingly, having the common partial derivative, the equation can be solved for Mhip.

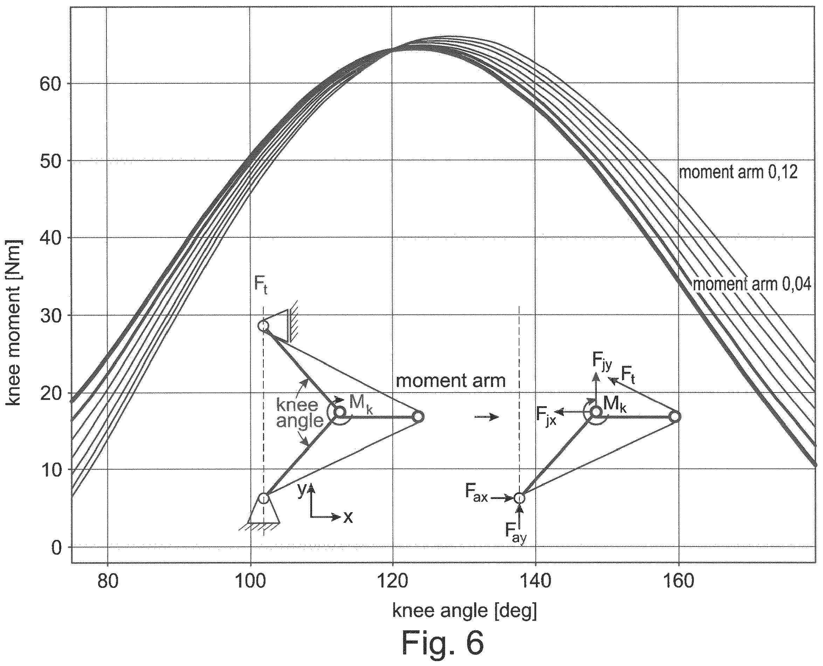

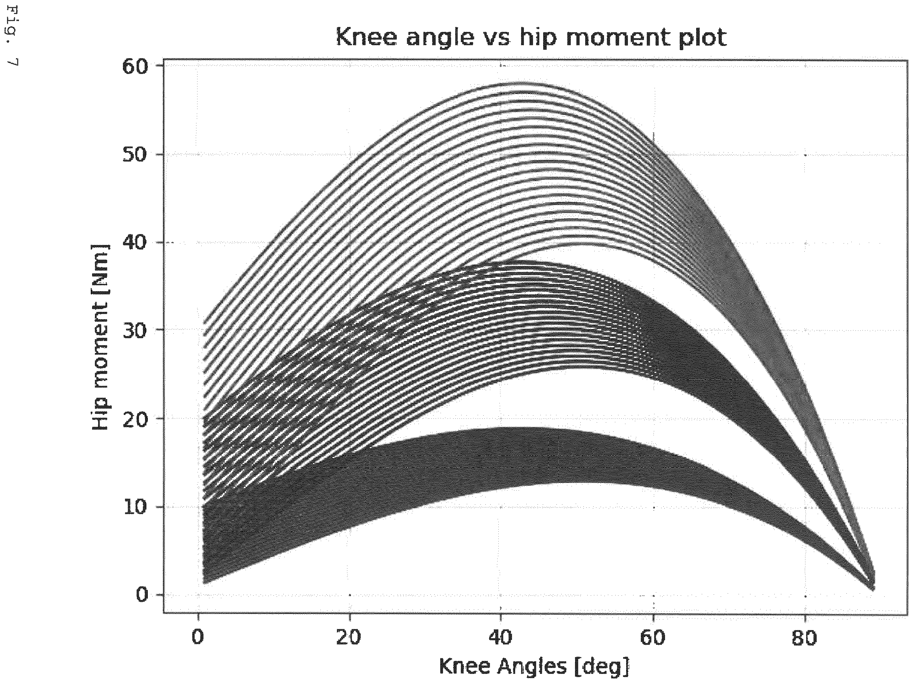

It can be deduced that the size of the KMA influences the moment generated around the hip as shown in Fig. 6 and 7, representing knee moment vs knee angle generated at different knee angles, showing the impact of different moment arms and hip mopment vs. knee angle for different moment arms respectively.

The wearable assistive device assists in a manner particularly useful for a large number of users. To understand this, it is important to realize that in walking, different phases can be differentiated, i.e. stance or swing phase, to provide the optimal support to the user. During stance phase the leg carries the user's weight and a closed kinematic chain is created. Once the foot is lifted from the ground and the leg is swinging forward, it acts like a pendulum swinging around the hip joint and knee joint. In contrast, during swing phase, there is no fixation available and the extension support of the knee is mainly dependent on the moment arm anterior to the knee joint.

Also, during movements with the foot firm on the ground, the muscles in the human body generate an extension moment that moves the entire body mass (e.g. walking, stair climbing, standing up from a chair). The extension pattern can be supported by a reaction force Freaction (cf. Fig. 1) due to the routing of the tendon (Ft).

In the arrangement shown, the knee is "pushed" posteriorly, generating moments that depend on the length of the shank and thigh segments rather than on the artificial moment arm that is anterior to the knee joint. As these moment arms are very large, they have a high influence on movement or posture, given that length shank and thigh element = app. 0.245*body height.

This is helpful, as the length of the artificial knee moment arm is a limiting factor of the practicability in everyday life scenarios. In the present invention, any artificial moment arm can be minimized, as the impact in supporting leg extension is small.

Also, the more the knee is bent, the more force is typically needed to assist the user in the extension movement, while less support is usually needed when the person is standing with straight legs and the tendon angles are bigger.

In the present invention, body positions with bent knees increase Freaction due to the sharp tendon angle around the knee. This can be seen in the generated knee moment. Especially when the leg is bent, larger moments are generated with smaller moment arms.

Only when the leg is almost straight, configurations with large moment arms generate larger knee moments, but this can be compensated by higher tendon forces. In contrast, during standing, the support moments required are low since the body weight is balanced in a stable configuration.

In view of this, Fig. 1 shows how Freaction can be used in an embodiment to support knee extension. By creating the reaction forces, the rigid elements attached to the thigh and shank rotate around R1 and R2. The straps 3 and 4 counteract the generated forces and transmit these forces to the segments of the user's limb. Straps 1 and 2 are making use of the conical limb shapes to prevent upwards and downwards shifting of the mechanical structure.

Now, since the forces are only transmitted laterally, the whole structure would tend to rotate around the vertical limb axis due to Freaction and rrot (Figure 1). The invention suggest that this can be prevented by suitable counter-torsion means, e.g. a snug fit (induced e.g. by a self-tightening thigh strap) or by changing the tendon routing such that rrot is minimized (angle β).

As the application of forces can be easily timed appropriately using a suitable control mechanism, for example a control mechanism implementing a model as described above, several benefits to a user to the knee joint can be implemented when applied, namely at least assistance in extension during the early stance phase of the gait cycle or during a sit-to-stand transition - becoming effectively an additional external muscle during the concentric knee extension movement; and knee stabilization during the mid-stance phase of the gait cycle or during standing, preventing the knee from collapsing

Support for the knee extension can be implemented such that a component positioned on the distal lateral and anterior sides of the thigh and a similar component positioned on the proximal anterior and lateral sides of the shank is provided. More specifically, the anterior thigh component shall rest on the posterior part of the quadriceps muscle, just above the quadriceps tendon. Similarly, the anterior part of the shank piece shall cover the area around tibial tubercle. To keep the piece attached to the leg segments, a strap system shall be used. The straps shall extend from the anterior parts of the two components and loop around the segments with an attachment point on posterior - lateral side of the stiff structures.

As disclosed, the two aforementioned structures are laterally connected by a hinge joint whose center of rotation generally aligns with that of the knee joint, noting that such general alignment need only be approximate. The two sections of the hinge joint extend but slightly from the two leg segment parts of the mechanism, as described above. The hinge joint has but a small protrusion that routes the tendon and extends anteriorly from the thigh half of the joint axis when viewed in sagittal plane. The tendon then continues from the hinge joint laterally over the thigh structure, posteriorly over the thigh (through a structure that sits on the thigh straps) and is attached to a force producing device such as a motor energized in accordance with control signals from a suitable control.

It is noted that a few rules of force flow preferably are followed around the knee joint in order to only induce a beneficial stabilization force in the system,: When viewed in the frontal plane, the path of the force transmitting member preferably lies collinearly with the lateral edge of the knee joint. This ensures that no rotation of the structure takes place in the frontal plane. By a tight fight of the thigh lpate, this can be easily warranted (cf. Fig. 1, anchor straps). Additionally and/or alternatively, a change in routing can be implemented to help in a compensation of forces (cf. Fig. 1, alternative path using $\beta$)

Furthermore, whereas - as has been described- to ensure that a large variety of user sizes can be accomodated by a single mechanism or very few parts, some degree of flexibility in the thigh and shank anterior component is typically provided. In view of the fact that this might lead to torsion due to the moment arm between the anterior pivot points of the two halves and the knee-aligned joint), that is relative torsional deformation occuring between the shank and thigh parts of the mechanism when tensile forces are applied to the tendon, the tendon as force transmitting member is typically and preferably routed from the hinge joint towards the anterior central point of the stiff thigh component

Also, the posterior routing is preferably guided around the thigh area through a semi-stiff member that attaches in a fixed manner to the thigh straps. This ensures that the force is transmitted in line with the humeral joint, when viewed in the frontal plane, ensuring that no hip abduction moment is induced.

Accordingly, due to the mechanical arrangement and the control thereof, moment arms required that exploit a closed kinematic chain and thus, the device of the present invention in particlar to be worn underneath clothes.

Also, high tangential forces are prevented by the small moment arm and the pivot points R1 and R2. Counteracting normal forces or pressure is generated by the corresponding straps (Figure 1), also preventing the structure from slipping upwards. It is to be noted that the length of the limb segment components preferably is at least 4 times bigger than the distance of the tendon to the center of Rotation (CoR). This reduces the pressure created by the strap. In addition, rotation around y-axis can prevented, inter alia due to a self-tightening mechanism (cf. anchor strap of Fig.1) and an additional force routing that minimizes rrot using the angle $\beta$.

It is noted that despite transmission of high forces, the unilateral knee joint of the present invention provides inherent safety since in contrast to other devices known it does not lock the spanned joint any longer once the kinematic chain is opened (e.g. foot is lifted from the ground.

Therefore, as can be seen from the above text, the arrangement of the present invention is advantageous compared to designs that require almost perfect alignment with the knee joint (e.g. exoskeletons).

The working principle of a self-tensioning strap as one example for counter-torsion means according to the present invention will now be explained in more detail with reference to Figs. 9-11. In order to visually display the functionalities of the counter-torsional mechanism and the self-tightening mechanism consider the example where the force is routed from the medial lower-back region of the user to the proximal lateral side of the thigh brace (from where it may then be further routed over the knee moment arm mechanism to the shank piece of the brace).

Fig. 9 shows a frontal plane view of such a thigh brace having a solid plate 2 of a wearable device according to a preferred embodiment of the present invention in order to display the transverse, sagittal and frontal planes.

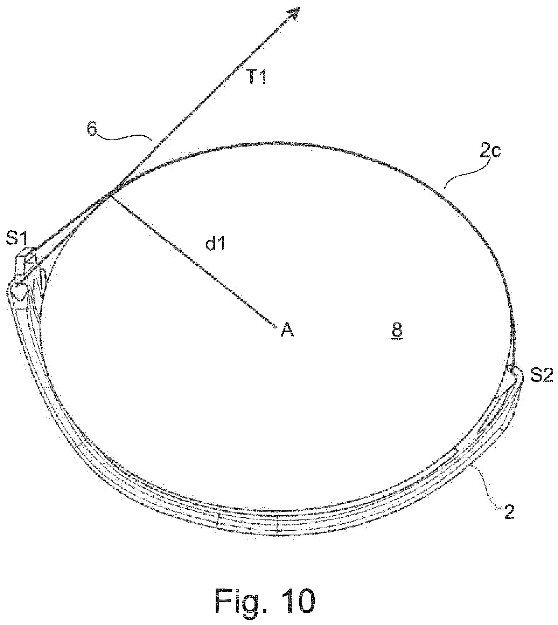

Fig. 10 schematically shows the moments generated within the transverse plane (through a cross-section of the user's thigh 8) without self-tensioning strap: if no counter-torsion means are provided, a moment Ma is generated in the transverse plane around the center A of the user's thigh 8. This moment Ma can be expressed in terms of the tension force T1 generated in the tendon 6 times the distance dl from the centre A of the user's thigh 8 in the transversal plane: Ma = d1 * T1

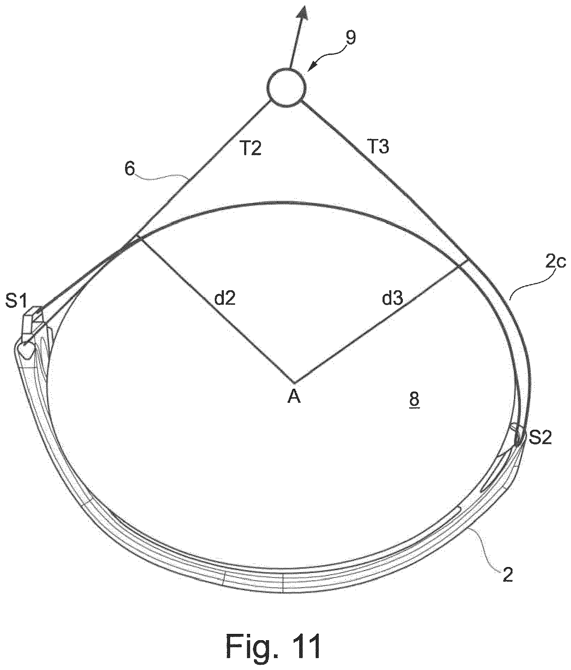

If the proximal thigh strap 2c extending in the thigh brace shown in Fig. 10 from a first anchoring point S1 of the stiff brace structure 2, i.e. the solid plate 2, to a second anchoring point S2 of the solid plate 2 is then further extended from the second anchoring point S2 to interface the tensioned tendon 6 at interface or connection 9 (in order to act as a pulley), a looped strap 2c is formed and the diagram shown in Fig. 11 can be generated.

As the tension in the tendon 6 is increased, the resultant tension force component is generated within the looped strap 2c. This force provides a "self-tensioning" mechanism for the proximal thigh strap 2c. The higher the tension force in the tendon 6, the more apparent is the "self-tensioning" in the thigh proximal strap 2c as its internal tension force (T3) is linearly proportional to the tension force of the tendon 6. The looped strap 2c now generates a counter-torsional moment around the user's center A of the thigh 8. The net moment around point A in the transversal plane can then be calculated as:

$$Ma = d2 * T2 - d3 * T3$$

**Claims**

1. A wearable assistive device (1) comprising first and second parts (2, 3), wearable proximal and distal of a limb joint (4) respectively, the first and second parts (2, 3) having an articulated interconnection (5) and a tendon interconnection (6), guided from the first part (2) to the second part (3) and adapted to exert an assistive force on the limb joint (4) when tensioned;

    wherein the tendon interconnection (6) is provided unilaterally, on the lateral side of the limb joint (4);
    wherein counter-torsion means (7) adapted to generate forces counteracting torsion of the first and second wearable parts (2, 3) relative to the limb on tensioning the tendon interconnection (6) are provided;
    wherein each of the first and second parts (2, 3) comprises a solid plate (2) adapted to be nestled in use to the limb; **characterised in that** the articulated interconnection (5) is provided unilaterally, on the lateral side of the limb joint (4) and
    the counter-torsion means (7) comprises routing means provided to route the tendon interconnection (6) from a hinge joint (5) between the two plates (2, 3) towards an anterior central point of the proximal plate (2).

2. The wearable assistive device according to claim 1, wherein a motor is provided adapted to tension the tendon interconnection (6), in particular a DC motor.

3. The wearable assistive device according to any of the preceding claims, wherein a control is provided to tension the tendon (6) in a manner stabilizing a posture and/or in a manner supporting an extension of the limb.

4. The wearable assistive device according to any of the preceding claims, wherein the first and second parts (2, 3) are adapted to be wearable proximal and distal of a knee joint (4) and the assistive device (1) is adapted to assist in stance stabilization and/or leg extension.

5. The wearable assistive device according to any of the preceding claims, wherein at least one and preferably both of the first and second parts (2, 3) comprises a solid plate (2, 3) adapted to be nestled in use to the limb and a strap arrangement (2a, 2b, 2c, 3a, 3b) comprising one or more straps (2a, 2b, 2c, 3a, 3b) adapted for attaching the plate (2, 3) on the limb.

6. The wearable assistive device according to claim 5, wherein the strap arrangement (2a, 2b, 2c, 3a, 3b) is adapted for attaching the plate (2, 3) on the limb such that at least one strap (2a, 2b, 2c, 3a, 3b) extends from one side of the plate (2, 3), around a posterior part of the limb and to an opposite side of the plate (2, 3).

7. The wearable assistive device according to claim 5 or 6, wherein the strap arrangement (2a, 2b, 2c, 3a, 3b) is adapted for attaching the plate (2) on the limb such that at least one strap (2c) is self-tensioning so as to implement a counter-torsion means.

8. The wearable assistive device according to claim 7, wherein the at least one self-tensioning strap (2c) is adapted to be guided from one side of the plate (2), around a posterior part of the limb, to an opposite side of the plate (2), and to the tendon interconnection (6).

9. The wearable assistive device according to claim 8, wherein the tension (6) generated within the self-tensioning strap (2c) upon tensioning the tendon interconnection (6), during use, generates a counter-torsional moment acting on the limb via the solid plate (2).

10. The wearable assistive device according to any of claims 5 to 9, wherein a tendon (6) of the tendon interconnection (6) is guided through a guiding member having a flexibility higher than that of the plate (2, 3) but lower than that of the tendon (6), the guiding member being fixedly attached to a strap of the strap arrangement (2a, 2b, 2c, 3a, 3b), in particular to a proximal thigh strap (2c).

11. The wearable assistive device according to the preceding claim, wherein the articulated interconnection (5) is provided in the form of a hinge joint (5) having a center of rotation aligned, in use, at least approximately with that of the knee joint (4), in particular to such a degree that any deviation is compensated in use by the compression of the device (1) and/or by the compression of the tissue of the user.

12. The wearable assistive device according to the preceding claim, wherein the hinge joint (5) is connected to a first and second plate (2, 3), wherein the most distal plate (3) is adapted to guide a tendon (6) of the tendon interconnection (6) and extends anteriorly from the joint axis when viewed in the sagittal plane.

13. The wearable assistive device according to any of the preceding claims, wherein the tendon interconnection (6) comprises a ribbon or a non-bowden cable, and in particular has a radius of curvature smaller than 1 cm, preferably smaller than 5 mm.

14. The wearable assistive device according to any of the preceding claims, wherein the tendon interconnection (6) is adapted to be routed such that the tendon (6) is guided laterally from a distal position below a knee joint (4) to a more medial position proximal of the knee joint (4), in particular at an angle BETA between 5° and 45°, preferably between 5° and 25° to a collinear path so as to implement a counter-torsion means.

**Patentansprüche**

1. Tragbare Unterstützungsvorrichtung (1) aufweisend ein erstes und zweites Teil (2, 3), die proximal bzw. distal eines Gliedgelenks (4) tragbar sind, wobei das erste und zweite Teil (2, 3) eine gelenkige gegenseitige Verbindung (5) und eine gegenseitige Sehnenverbindung (6) aufweisen, die von dem ersten Teil (2) zu dem zweiten Teil (3) geführt wird und angepasst ist, eine unterstützende Kraft auf das Gliedgelenk (4) auszuüben, wenn gespannt;

   wobei die gegenseitige Sehnenverbindung (6) unilateral an der lateralen Seite des Gliedgelenks (4) bereitgestellt ist;
   wobei ein Gegentorsionsmittel (7) bereitgestellt ist, das angepasst ist, beim Spannen der gegenseitigen Sehnenverbindung (6) Kräfte zu erzeugen, die Torsion des ersten und zweiten tragbaren Teils (2, 3) relativ zu dem Glied entgegenwirken;
   wobei sowohl das erste als auch das zweite Teil (2, 3) eine massive Platte (2) aufweist, die angepasst ist, sich in Gebrauch an das Glied anzuschmiegen;
   **dadurch gekennzeichnet, dass** die gelenkige gegenseitige Verbindung (5) unilateral an der lateralen Seite des Gliedgelenks (4) bereitgestellt ist und das Gegentorsionsmittel (7) ein Lenkungsmittel aufweist, das bereitgestellt ist, um die gegenseitige Sehnenverbindung (6) von einem Scharniergelenk (5) zwischen den zwei Platten (2, 3) hin zu einem vorderen zentralen Punkt der proximalen Platte (2) zu lenken.

2. Tragbare Unterstützungsvorrichtung nach Anspruch 1, wobei ein zum Spannen der gegenseitigen Sehnenverbindung (6) angepasster Motor insbesondere ein Gleichstrommotor, bereitgestellt ist.

3. Tragbare Unterstützungsvorrichtung nach einem der vorstehenden Ansprüche, wobei eine Steuerung bereitgestellt ist, um die Sehne (6) zu spannen auf eine Weise, die eine Haltung stabilisiert, und/oder auf eine Weise, die eine Streckung des Glieds unterstützt.

4. Tragbare Unterstützungsvorrichtung nach einem der vorstehenden Ansprüche, wobei das erste und zweite Teil (2, 3) angepasst sind, proximal und distal eines Kniegelenks (4) tragbar zu sein, und die Unterstützungsvorrichtung (1) angepasst ist, bei Haltungsstabilisierung und/oder Beinstreckung zu unterstützen.

5. Tragbare Unterstützungsvorrichtung nach einem der vorstehenden Ansprüche, wobei mindestens eines und vorzugsweise beide des ersten und zweiten Teils (2, 3) eine massive Platte (2, 3), die angepasst ist, sich in Gebrauch an das Glied anzuschmiegen, und eine Gurtanordnung (2a, 2b, 2c, 3a, 3b), die einen oder mehrere Gurte (2a, 2b, 2c, 3a, 3b) aufweist, die angepasst sind, die Platte (2, 3) an dem Glied zu befestigen, aufweist/aufweisen.

**6.** Tragbare Unterstützungsvorrichtung nach Anspruch 5, wobei die Gurtanordnung (2a, 2b, 2c, 3a, 3b) angepasst ist, die Platte (2, 3) derart an dem Glied zu befestigen, dass sich mindestens ein Gurt (2a, 2b, 2c, 3a, 3b) von einer Seite der Platte (2, 3), um einen hinteren Teil des Glieds und zu einer gegenüberliegenden Seite der Platte (2, 3) erstreckt.

**7.** Tragbare Unterstützungsvorrichtung nach Anspruch 5 oder 6, wobei die Gurtanordnung (2a, 2b, 2c, 3a, 3b) angepasst ist, die Platte (2) derart an dem Glied zu befestigen, dass mindestens ein Gurt (2c) selbstspannend ist, um so ein Gegentorsionsmittel zu verwirklichen.

**8.** Tragbare Unterstützungsvorrichtung nach Anspruch 7, wobei der mindestens eine selbstspannende Gurt (2c) angepasst ist, von einer Seite der Platte (2), um einen hinteren Teil des Glieds zu einer gegenüberliegenden Seite der Platte (2) und zu der gegenseitigen Sehnenverbindung (6) geführt zu werden.

**9.** Tragbare Unterstützungsvorrichtung nach Anspruch 8, wobei die Spannung (6), die während des Gebrauchs beim Spannen der gegenseitigen Sehnenverbindung (6) in dem selbstspannenden Gurt (2c) erzeugt wird, ein Gegentorsionsmoment erzeugt, das über die massive Platte (2) auf das Glied wirkt.

**10.** Tragbare Unterstützungsvorrichtung nach einem der Ansprüche 5 bis 9, wobei eine Sehne (6) der gegenseitigen Sehnenverbindung (6) durch ein Führungselement geführt wird, das eine Flexibilität hat, die höher als die der Platte (2, 3), aber geringer als die der Sehne (6) ist, wobei das Führungselement fest an einem Gurt der Gurtanordnung (2a, 2b, 2c, 3a, 3b), insbesondere an einem proximalen Oberschenkelgurt (2c), angebracht ist.

**11.** Tragbare Unterstützungsvorrichtung nach dem vorstehenden Anspruch, wobei die gelenkige gegenseitige Verbindung (5) in Form eines Scharniergelenks (5) bereitgestellt ist, dessen Drehzentrum in Gebrauch mindestens ungefähr mit dem des Kniegelenks (4) ausgerichtet ist, insbesondere in einem solchen Maß, dass in Gebrauch jede Abweichung durch die Kompression der Vorrichtung (1) und/oder durch die Kompression des Gewebes des Benutzers kompensiert wird.

**12.** Tragbare Unterstützungsvorrichtung nach dem vorstehenden Anspruch, wobei das Scharniergelenk (5) mit einer ersten und zweiten Platte (2, 3) verbunden ist, wobei die distalste Platte (3) angepasst ist, eine Sehne (6) der gegenseitigen Sehnenverbindung (6) zu führen, und sich anterior von der Gelenkachse erstreckt, wenn in der Sagittalebene betrachtet.

**13.** Tragbare Unterstützungsvorrichtung nach einem der vorstehenden Ansprüche, wobei die gegenseitige Sehnenverbindung (6) ein Band oder einen Nicht-Bowdenzug aufweist und insbesondere einen Krümmungsradius aufweist, der kleiner als 1cm, vorzugsweise kleiner als 5mm, ist.

**14.** Tragbare Unterstützungsvorrichtung nach einem der vorstehenden Ansprüche, wobei die gegenseitige Sehnenverbindung (6) angepasst ist, derart gelenkt zu werden, dass die Sehne (6) lateral von einer distalen Position unterhalb eines Kniegelenks (4) zu einer mehr medialen Position proximal des Kniegelenks (4), insbesondere in einem Winkel BETA zwischen 5° und 45°, vorzugsweise zwischen 5° und 25°, zu einem kollinearen Pfad, geführt wird, um so ein Gegentorsionsmittel zu verwirklichen.

**Revendications**

**1.** Dispositif d'assistance portable (1) comprenant des première et seconde parties (2, 3) pouvant être respectivement portées à proximité et à distance d'une articulation de membre (4), les première et seconde parties (2, 3) ayant une interconnexion articulée (5) et une interconnexion de tendon (6) guidées de la première partie (2) à la seconde partie (3) et adaptées pour exercer une force d'assistance sur l'articulation de membre (4) lorsqu'elles sont tendues ;

dans lequel l'interconnexion de tendon (6) est prévue de manière unilatérale, sur le côté latéral de l'articulation de membre (4) ;
dans lequel les moyens de contre-torsion (7) adaptés pour générer des forces contrecarrant la torsion des première et seconde parties (2, 3) pouvant être portées par rapport au membre en tendant l'interconnexion de tension (6), sont prévus ;
dans lequel chacune des première et seconde parties (2, 3) comprend une plaque solide (2) adaptée pour être nichée, à l'usage, dans le membre ; **caractérisé en ce que** l'interconnexion articulée (5) est prévue de manière unilatérale, sur le côté latéral de l'articulation de membre (4), et

le moyen de contre-torsion (7) comprend un moyen d'acheminement prévu pour acheminer l'interconnexion de tendon (6) d'une articulation à charnière (5) entre les deux plaques (2, 3) vers un point central antérieur de la plaque proximale (2).

2.  Dispositif d'assistance portable selon la revendication 1, dans lequel un moteur est prévu en étant adapté pour tendre l'interconnexion de tendon (6), en particulier un moteur CC.

3.  Dispositif d'assistance portable selon l'une quelconque des revendications précédentes, dans lequel une commande est prévue pour tendre le tendon (6) afin de stabiliser une posture et/ou afin de supporter une extension du membre.

4.  Dispositif d'assistance portable selon l'une quelconque des revendications précédentes, dans lequel les première et seconde parties (2, 3) sont adaptées pour être portées à proximité et à distance d'une articulation de genou (4) et le dispositif d'assistance (1) est adapté pour aider à stabiliser la position des pieds et/ou l'extension de jambe.

5.  Dispositif d'assistance portable selon l'une quelconque des revendications précédentes, dans lequel au moins l'une et de préférence les deux des première et seconde parties (2, 3) comprend (comprennent) une plaque solide (2, 3) adaptée pour être nichée, à l'usage, dans le membre et un agencement de sangle (2a, 2b, 2c, 3a, 3b) comprenant une ou plusieurs sangles (2a, 2b, 2c, 3a, 3b) adaptées pour fixer la plaque (2, 3) sur le membre.

6.  Dispositif d'assistance portable selon la revendication 5, dans lequel l'agencement de sangle (2a, 2b, 2c, 3a, 3b) est adapté pour fixer la plaque (2, 3) sur le membre de sorte qu'au moins une sangle (2a, 2b, 2c, 3a, 3b) s'étend à partir d'un côté de la plaque (2, 3), autour d'une partie postérieure du membre et vers un côté opposé de la plaque (2, 3).

7.  Dispositif d'assistance portable selon la revendication 5 ou 6, dans lequel l'agencement de sangle (2a, 2b, 2c, 3a, 3b) est adapté pour fixer la plaque (2) sur le membre de sorte qu'au moins une sangle (2c) se tend automatiquement afin de mettre en oeuvre un moyen de contre-torsion.

8.  Dispositif d'assistance portable selon la revendication 7, dans lequel la au moins une sangle à tension automatique (2c) est adaptée pour être guidée d'un côté de la plaque (2), autour d'une partie postérieure du membre, vers un côté opposé de la plaque (2) et vers l'interconnexion de tendon (6).

9.  Dispositif d'assistance portable selon la revendication 8, dans lequel la tension (6) générée dans la sangle à tension automatique (2c) suite à la tension de l'interconnexion de tendon (6), pendant l'utilisation, génère un moment de contre-torsion agissant sur le membre via la plaque solide (2).

10.  Dispositif d'assistance portable selon l'une quelconque des revendications 5 à 9, dans lequel un tendon (6) de l'interconnexion de tension (6) est guidé par le biais d'un élément de guidage présentant une flexibilité supérieure à celle de la plaque (2, 3) mais inférieure à celle du tension (6), l'élément de guidage étant fixement fixé à une sangle de l'agencement de sangle (2a, 2b, 2c, 3a, 3b), en particulier à une sangle de cuisse proximale (2c).

11.  Dispositif d'assistance portable selon la revendication précédente, dans lequel l'interconnexion articulée (5) est prévue sous la forme d'une articulation à charnière (5) ayant un centre de rotation aligné, à l'usage, au moins approximativement avec celui de l'articulation de genou (4), en particulier à un degré tel que toute déviation est compensée, à l'usage, par la compression du dispositif (1) et/ou par la compression du tissu de l'utilisateur.

12.  Dispositif d'assistance portable selon la revendication précédente, dans lequel l'articulation à charnière (5) est raccordée à une première et à une seconde plaque (2, 3), dans lequel la plaque la plus distale (3) est adaptée pour guider un tendon (6) de l'interconnexion de tendon (6) et s'étend de manière antérieure à partir de l'axe d'articulation lorsqu'il est observé dans le plan sagittal.

13.  Dispositif d'assistance portable selon l'une quelconque des revendications précédentes, dans lequel l'interconnexion de tension (6) comprend un ruban ou un câble autre qu'un câble Bowden, et en particulier a un rayon de courbure inférieur à 1 cm, de préférence inférieur à 5 mm.

14.  Dispositif d'assistance portable selon l'une quelconque des revendications précédentes, dans lequel l'interconnexion de tension (6) est adaptée pour être acheminée de sorte que le tendon (6) est guidé latéralement à partir d'une

EP 3 793 493 B1

position distale au-dessous d'une articulation de genou (4) jusqu'à une position plus médiale à proximité de l'articulation de genou (4), en particulier à un angle BETA compris entre 5° et 45°, de préférence compris entre 5° et 25° jusqu'à une trajectoire colinéaire afin de mettre en oeuvre un moyen de contre-torsion.

Fig. 1

FRONT

SIDE

BACK

$F_{tendon}$, $F_{reaction}$, CoR, $r_{rot}$, $\beta$, C

$p_{c,t}$, $x_t$, $p_{c,s}$, $x_s$, $R_1$, $R_2$, $\omega$

anchor-strap

EP 3 793 493 B1

Fig. 2

2d

2f

2

2e1 2e2 2e3

5

Fig. 3

Fig. 4

Fig. 5

Fig. 6

Fig. 7

Fig. 8

25

Transverse plane

Sagittal plane

2

Frontal plane

Fig. 9

Fig. 10

Fig. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4100918 A
- KR 1020120062375 A
- EP 0205785 B1
- EP 0564734 A1
- US 6635024 B2
- US 9597217 B2
- WO 2015157731 A
- WO 2018023109 A1
- WO 2018122106 A1
- US 2010056970 A1
- US 2006206043 A1
- US 2018000623 A1
- WO 2018122106 A
- DE 102018215163